# EUROPÄISCHE PATENTSCHRIFT

(11) **EP 1 711 469 B1**
(45) Veröffentlichungstag und Bekanntmachung des Hinweises auf die Patenterteilung: **15.07.2009**
(21) Anmeldenummer: 05700962.3
(22) Anmeldetag: 15.01.2005
(51) Int. Cl.: C07D 221/18

(54) **VERFAHREN ZUR HERSTELLUNG VON TERRYLEN-3,4:11,12-TETRACARBONSÄUREDIIMIDEN DURCH DIREKTSYNTHESE**
METHOD FOR THE PRODUCTION OF TERYLENE-3,4:11,12-TETRACARBOXYDIIMIDES BY DIRECT SYNTHESIS
PROCEDE DE PRODUCTION DE DIIMIDES DE TERYLENE-3,4:11,12-ACIDE TETRACARBOXYLIQUE PAR SYNTHESE DIRECTE

(30) Priorität: 23.01.2004 DE 102004003734
(43) Veröffentlichungstag der Anmeldung: 18.10.2006
(73) Patentinhaber: BASF SE, 67056 Ludwigshafen (DE); Max-Planck-Gesellschaft zur Förderung der Wissenschaften e.V., 80539 München (DE)
(72) Erfinder: KÖNEMANN, Martin, 68163 Mannheim (DE); BÖHM, Arno, 68305 Mannheim (DE); HELFER, Willi, 67159 Friedelsheim (DE); ROMEIS, Jürgen, 67069 Ludwigshafen (DE); QU, Jianqiang, 68169 Mannheim (DE); MÜLLEN, Klaus, 50939 Köln (DE)
(86) Internationale Anmeldenummer: PCT/EP2005/000378
(87) Internationale Veröffentlichungsnummer: WO 2005/070895

(56) Entgegenhaltungen:
- DE-A1- 19 512 773
- HOLTRUP F O ET AL: "TERRYLENIMIDES: NEW NIR FLUORESCENT DYES" CHEMISTRY - A EUROPEAN JOURNAL, VCH PUBLISHERS, US, Bd. 3, Nr. 2, 1997, Seiten 219-225, XP000931226 ISSN: 0947-6539 in der Anmeldung erwähnt
- WEIL TANJA ET AL: "Synthesis and characterization of dendritic multichromophores based on rylene dyes for vectorial transduction of excitation energy" CHEMISTRY - A EUROPEAN JOURNAL, VCH PUBLISHERS, US, Bd. 10, Nr. 6, 2004, Seiten 1398-1414, XP002316375 ISSN: 0947-6539
- LANGHALS H ET AL: "A Two-Step Synthesis of Quaterrylenetetracarboxylic Bisimides-Novel NIR Fluorescent Dyes" TETRAHEDRON LETTERS, ELSEVIER SCIENCE PUBLISHERS, AMSTERDAM, NL, Bd. 36, Nr. 36, 4. September 1995 (1995-09-04), Seiten 6423-6424, XP004027248 ISSN: 0040-4039

## Beschreibung

Die vorliegende Erfindung betrifft ein neues Verfahren zur Herstellung von Terrylen-3,4:11,12-tetracarbonsäurediimiden der allgemeinen Formel I 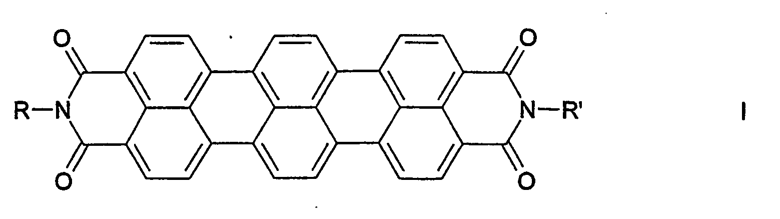 in der die Variablen folgende Bedeutung haben:
- R, R': unabhängig voneinander Wasserstoff; C₁-C₃₀-Alkyl, dessen Kohlenstoffkette durch eine oder mehrere Gruppierungen -O-, -S-, -NR¹-, -CO- und/oder -SO₂- unterbrochen sein kann und das durch Cyano, C₁-C₆-Alkoxy, Aryl, das durch C₁-C₁₈-Alkyl oder C₁-C₆-Alkoxy substitu- iert sein kann, und/oder einen über ein Stickstoffatom gebundenen 5- bis 7- gliedrigen heterocyclischen Rest, der weitere Heteroatome enthalten und a- romatisch sein kann, ein- oder mehrfach substituiert sein kann; C₅-C₈-Cycloalkyl, dessen Kohlenstoffgerüst durch eine oder mehrere Gruppie- rungen -O-, -S- und/oder -NR¹- unterbrochen und/oder das durch C₁-C₆-Alkyl ein- oder mehrfach substituiert sein kann; Aryl oder Hetaryl, das durch C₁-C₁₈-Alkyl, C₁-C₆-Alkoxy, Cyano, Halogen, -CONHR² und/oder Aryl- oder Hetarylazo, das jeweils durch C₁-C₁₀-Alkyl, C₁-C₆-Alkoxy oder Cyano substituiert sein kann, ein- oder mehrfach substitu- iert sein kann;
- R¹: Wasserstoff oder C₁-C₆-Alkyl;
- R²: Wasserstoff; C₁-C₁₈-Alkyl; Aryl oder Hetaryl, das jeweils durch C₁-C₆-Alkyl, C₁-C₆-Alkoxy, Halogen, Hydroxy, Carboxy oder Cyano substituiert sein kann.

Terrylen-3,4:11,12-tetracarbonsäurediimide eignen sich bekanntermaßen als Pigmente und Fluoreszenzfarbstoffe mit Absorption im langwellig roten und Fluoreszenzemission im langwellig roten bis nahinfraroten Bereich des elektromagnetischen Spektrums.

In Chem. Eur. J. 3, S. 219 - 225 (1997) ist ein Verfahren zu ihrer Herstellung beschrieben, das von 5-Bromacenaphthenchinon ausgeht und eine Vielzahl von Reaktionsschritten umfaßt: Ketalisierung, Überführung in eine Boronsäure, Umsetzung mit einem 9-Bromperylen-3,4-dicarbonsäureimid in einer Suzuki-Kupplungsreaktion zu einem 9-(4-Acenaphthochinonyl)perylen-3,4-dicarbonsäureimid, Oxidation zum Tetracarbonsäureimidanhydrid, Imidierung zum Diimid und Cyclodehydrierung zum Terrylen-3,4:11,12-tetracarbonsäurediimid.

N,N'-Dialkylsubstituierte Terrylen-3,4:11,12-tetracarbonsäurediimide sind gemäß Heterocycles 56, S. 331 - 340 (2002) zugänglich, indem ein N-Alkyl-9-bromperylen-3,4-dicarbonsäureimid zum 9-Tributylzinnderivat umgesetzt wird, das dann mit einem N-Alkyl-4-Halogennaphthalin-1,8-dicarbonsäureimid zum entsprechenden 9-(4-Naphthalin-1,8-dicarbonsäureimid)perylen-3,4-dicarbonsäureimid gekuppelt wird, aus dem wiederum durch Cyclodehydrierung das Terrylen-3,4:11,12-tetracarbonsäurediimid hergestellt wird.

Die bekannten Herstellungsverfahren haben eine Reihe von Nachteilen: Es werden toxische Zinnverbindungen und/oder starke Basen in großen Mengen eingesetzt, die Reaktionszeiten sind sehr lang, und/oder die Gesamtausbeute liegt bei unter 50%.

Der Erfindung lag daher die Aufgabe zugrunde, diesen Nachteilen abzuhelfen und ein Verfahren bereitzustellen, das die Herstellung von Terrylen-3,4:11,12-tetracarbonsäurediimiden auf vorteilhafte, wirtschaftliche Weise ermöglicht.

Demgemäß wurde ein Verfahren zur Herstellung von Terrylen-3,4:11,12 tetracarbonsäurediimiden der allgemeinen Formel I 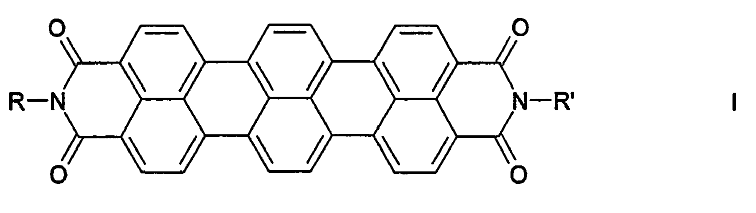 in der die Variablen die eingangs angegebene Bedeutung haben, gefunden, welches dadurch gekennzeichnet ist, daß man ein Perylen-3,4-dicarbonsäureimid der allgemeinen Formel II 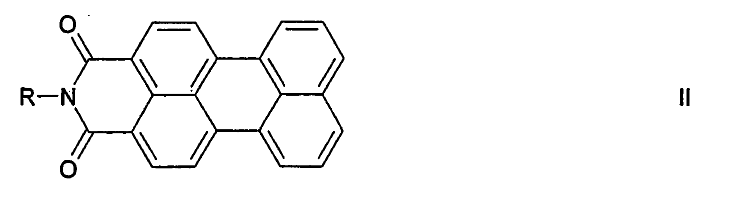 in Gegenwart eines basenstabilen, hochsiedenden, organischen Lösungsmittels und einer alkali- oder erdalkalimetallhaltigen Base mit einem Naphthalin-1,8-dicarbonsäureimid der allgemeinen Formel III 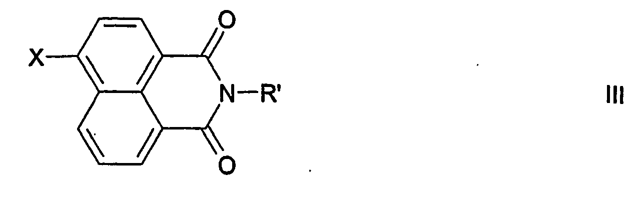 in der X Wasserstoff, Brom oder Chlor bedeutet, umsetzt.

Alle in den Formeln I bis III auftretenden Alkylgruppen können geradkettig oder verzweigt sein. Wenn die Alkylgruppen substituiert sind, tragen sie in der Regel 1 oder 2 Substituenten.

Cycloalkylgruppen und aromatische Reste, die substituiert sind, können im allgemeinen bis zu 3, bevorzugt 1 oder 2, der genannten Substituenten aufweisen.

Als Beispiele für geeignete Reste R, R', R¹, R² und R³ (bzw. für deren Substituenten) seien im einzelnen genannt:
Methyl, Ethyl, Propyl, Isopropyl, Butyl, Isobutyl, tert.-Butyl, Pentyl, Isopentyl, Neopentyl, tert.-Pentyl, Hexyl, 2-Methylpentyl, Heptyl, 1-Ethylpentyl, Octyl, 2-Ethylhexyl, Isooctyl, Nonyl, Isononyl, Decyl, Isodecyl, Undecyl, Dodecyl, Tridecyl, Isotridecyl, Tetradecyl, Pentadecyl, Hexadecyl, Heptadecyl, Octadecyl, Nonadecyl und Eicosyl (die obigen Bezeichnungen Isooctyl, Isononyl, Isodecyl und Isotridecyl sind Trivialbezeichnungen und stammen von den nach der Oxosynthese erhaltenen Alkoholen);
Methoxymethyl, 2-Methoxyethyl, 2-Ethoxyethyl, 2-Propoxyethyl, 2-Isopropoxyethyl, 2-Butoxyethyl, 2- und 3-Methoxypropyl, 2- und 3-Ethoxypropyl, 2- und 3-Propoxypropyl, 2- und 3-Butoxypropyl, 2- und 4-Methoxybutyl, 2- und 4-Ethoxybutyl, 2- und 4-Propoxybutyl, 3,6-Dioxaheptyl, 3,6-Dioxaoctyl, 4,8-Dioxanonyl, 3,7-Dioxaoctyl, 3,7-Dioxanonyl, 4,7-Dioxaoctyl, 4,7-Dioxanonyl, 2- und 4-Butoxybutyl, 4,8-Dioxadecyl, 3,6,9-Trioxadecyl, 3,6,9-Trioxaundecyl, 3,6,9-Trioxadodecyl, 3,6,9,12-Tetraoxatridecyl und 3,6,9,12-Tetraoxatetradecyl;
Methylthiomethyl, 2-Methylthioethyl, 2-Ethylthioethyl, 2-Propylthioethyl, 2-Isopropylthioethyl, 2-Butylthioethyl, 2- und 3-Methylthiopropyl, 2- und 3-Ethylthiopropyl, 2- und 3-Propylthiopropyl, 2- und 3-Butylthiopropyl, 2- und 4-Methylthiobutyl, 2- und 4-Ethylthiobutyl, 2- und 4-Propylthiobutyl, 3,6-Dithiaheptyl, 3,6-Dithiaoctyl, 4,8-Dithianonyl, 3,7-Dithiaoctyl, 3,7-Dithianonyl, 2- und 4-Butylthiobutyl, 4,8-Dithiadecyl, 3,6,9-Trithiadecyl, 3,6,9-Trithiaundecyl, 3,6,9-Trithiadodecyl, 3,8,9,12-Tetrathiatridecyl und 3,6,9,12-Tetrathiatetradecyl;
2-Monomethyl- und 2-Monoethylaminoethyl, 2-Dimethylaminoethyl, 2- und 3- Dimethylaminopropyl, 3-Monoisopropylaminopropyl, 2- und 4-Monopropylaminobutyl, 2- und 4-Dimethylaminobutyl, 6-Methyl-3,6-diazaheptyl, 3,6-Dimethyl-3,6-diazaheptyl, 3,6-Diazaoctyl, 3,6-Dimethyl-3,6-diazaoctyl, 9-Methyl-3,6,9-triazadecyl, 3,6,9-Trimethyl-3,6,9-triazadecyl, 3,6,9-Triazaundecyl, 3,6,9-Trimethyl-3,6,9-triazaundecyl, 12-Methyl-3,6,9,12-tetraazatridecyl und 3,6,9,12-Tetramethyl-3,6,9,12-tetraazatridecyl;
Propan-2-on-1-yl, Butan-3-on-1-yl, Butan-3-on-2-yl und 2-Ethylpentan-3-on-1-yl;
2-Methylsulfonylethyl, 2-Ethylsulfonylethyl, 2-Propylsulfonylethyl, 2-Isopropylsulfonylethyl, 2-Butylsulfonylethyl, 2- und 3-Methylsulfonylpropyl, 2- und 3-Ethylsulfonylpropyl, 2- und 3-Propylsulfonylpropyl, 2- und 3-Butylsulfonylpropyl, 2- und 4-Methylsulfonylbutyl, 2- und 4-Ethylsulfonylbutyl, 2- und 4-Propylsulfonylbutyl und 4-Butylsulfonylbutyl;
Cyanomethyl, 2-Cyanoethyl, 3-Cyanopropyl, 2-Methyl-3-ethyl-3-cyanopropyl, 7-Cyano-7-ethylheptyl und 4,7-Dimethyl-7-cyanoheptyl;
Methoxy, Ethoxy, Propoxy, Isopropoxy, Butoxy, Isobutoxy, sec.-Butoxy, tert.-Butoxy, Pentoxy, Isopentoxy, Neopentoxy, tert.-Pentoxy und Hexoxy;
Carbamoyl, Methylaminocarbonyl, Ethylaminocarbonyl, Propylaminocarbonyl, Butylaminocarbonyl, Pentylaminocarbonyl, Hexylaminocarbonyl, Heptylaminocarbonyl, Octylaminocarbonyl, Nonylaminocarbonyl, Decylaminocarbonyl und Phenylaminocarbonyl;
Chlor, Brom und lod;
Phenylazo, 2-Napthylazo, 2-Pyridylazo und 2-Pyrimidylazo;
Phenyl, 1- und 2-Naphthyl, 2- und 3-Pyrryl, 2-, 3- und 4-Pyridyl, 2-, 4- und 5-Pyrimidyl, 3-, 4- und 5-Pyrazolyl, 2-, 4- und 5-Imidazolyl, 2-, 4- und 5-Thiazolyl, 3-(1,2,4-Triazyl), 2-(1,3,5-Triazyl), 6-Chinaldyl, 3-, 5-, 6- und 8-Chinolinyl, 2-Benzoxazolyl, 2-Benzothiazolyl, 5-Benzothiadiazolyl, 2- und 5-Benzimidazolyl und 1- und 5- Isochinolyl;
2-, 3- und 4-Methylphenyl, 2,3-, 2,4-, 2,5-, 3,5- und 2,6-Dimethylphenyl, 2,4,6-Trimethylphenyl, 2-, 3- und 4-Ethylphenyl, 2,3-, 2,4-, 2,5-, 3,5- und 2,6-Diethylphenyl, 2,4,6-Triethylphenyl, 2-, 3- und 4-Propylphenyl, 2,3-, 2,4-, 2,5-, 3,5- und 2,6-Dipropylphenyl, 2,4,6-Tripropylphenyl, 2-, 3- und 4-Isopropylphenyl, 2,3-, 2,4-, 2,5-, 3,5- und 2,6-Diisopropylphenyl, 2,4,6-Triisopropylphenyl, 2-, 3- und 4-Butylphenyl, 2,3-, 2,4-, 2,5-, 3,5- und 2,6-Dibutylphenyl, 2,4,6-Tributylphenyl, 2-, 3- und 4-Isobutylphenyl, 2,3-, 2,4-, 2,5-, 3,5- und 2,6-Diisobutylphenyl, 2,4,6-Triisobutylphenyl, 2-, 3- und 4-sec.-Butylphenyl, 2,3-, 2,4-, 2,5-, 3,5- und 2,6-Di-sec.-butylphenyl und 2,4,6-Tri-sec.-butylphenyl, 2-, 3- und 4-tert.-Butylphenyl, 2,3-, 2,4-, 2,5-, 3,5- und 2,6-Di-tert.-butylphenyl, 2,4,6-Tri-tert.-butylphenyl; 2-, 3- und 4-Methoxyphenyl, 2,3-, 2,4-, 2,5-, 3,5- und 2,6-Dimethoxyphenyl, 2,4,6-Trimethoxyphenyl, 2-, 3- und 4-Ethoxyphenyl, 2,3-, 2,4-, 2,5-, 3,5- und 2,6-Diethoxyphenyl, 2,4,6-Triethoxyphenyl, 2-, 3- und 4-Propoxyphenyl, 2,3-, 2,4-, 2,5-, 3,5- und 2,6-Dipropoxyphenyl, 2-, 3- und 4-Isopropoxyphenyl, 2,3-, 2,4-, 2,5-, 3,5- und 2,6-Diisopropoxyphenyl und 2-, 3- und 4-Butoxyphenyl; 2-, 3- und 4-Chlorphenyl, und 2,3-, 2,4-, 2,5-, 3,5- und 2,6-Dichlorphenyl; 2-, 3- und 4-Hydroxyphenyl und 2,3-, 2,4-, 2,5-, 3,5- und 2,6-Dihydroxyphenyl; 2-, 3- und 4-Cyanophenyl; 3- und 4-Carboxyphenyl; 3- und 4-Carboxamidophenyl, 3- und 4-N-Methylcarboxamidophenyl und 3- und 4-N-Ethylcarboxamidophenyl; 3- und 4-Acetylaminophenyl, 3- und 4-Propionylaminophenyl und 3- und 4-Butyrylaminophenyl; 3- und 4-N-Phenylaminophenyl, 3-und 4-N-(o-Tolyl)aminophenyl, 3- und 4-N-(m-Tolyl)aminophenyl und 3- und 4-N-(p-Tolyl)aminophenyl; 3- und 4-(2-Pyridyl)aminophenyl, 3- und 4-(3-Pyridyl)aminophenyl, 3-und 4-(4-Pyridyl)aminophenyl, 3- und 4-(2-Pyrimidyl)aminophenyl und 4-(4-Pyrimidyl)-aminophenyl;
4-Phenylazophenyl, 4-(1-Naphthylazo)phenyl, 4-(2-Naphthylazo)phenyl, 4-(4-Naphthylazo)phenyl, 4-(2-Pyridylazo)phenyl, 4-(3-Pyridylazo)phenyl, 4-(4-Pyridylazo)phenyl, 4-(2-Pyrimidylazo)phenyl, 4-(4-Pyrimidylazo)phenyl und 4-(5-Pyrimidylazo)phenyl;
Cyclopentyl, 2- und 3-Methylcyclopentyl, 2- und 3-Ethylcyclopentyl, Cyclohexyl, 2-, 3-und 4-Methylcyclohexyl, 2-, 3- und 4-Ethylcyclohexyl, 3- und 4-Propylcyclohexyl, 3- und 4-Isopropylcyclohexyl, 3- und 4-Butylcyclohexyl, 3- und 4-sec.-Butylcyclohexyl, 3- und 4-tert.-Butylcyclohexyl, Cycloheptyl, 2-, 3- und 4-Methylcycloheptyl, 2-, 3- und 4-Ethylcycloheptyl, 3- und 4-Propylcycloheptyl, 3- und 4-Isopropylcycloheptyl, 3- und 4-Butylcycloheptyl, 3- und 4-sec.-Butylcycloheptyl, 3- und 4-tert.-Butylcycloheptyl, Cyclooctyl, 2-, 3-, 4- und 5-Methylcyclooctyl, 2-, 3-, 4- und 5-Ethyicyclooctyl, 3-, 4- und 5-Propylcyclooctyl, 2-Dioxanyl, 4-Morpholinyl, 2- und 3-Tetrahydrofuryl, 1-, 2- und 3-Pyrrolidinyl und 1-, 2-, 3- und 4-Piperidyl.

Mit Hilfe des erfindungsgemäßen Verfahrens können die Terrylen-3,4:11,12-tetracarbonsäurediimide I durch Umsetzung eines Perylen-3,4-dicarbonsäureimids II mit einem Naphthalin-1,8-dicabonsäureimid III in einem Schritt hergestellt werden.

Die erfindungsgemäße Umsetzung wird in Gegenwart eines basenstabilen, hochsiedenden, organischen Lösungsmittels und einer Alkalimetallbase durchgeführt.

Als Edukt kann dabei sowohl in 4-Position halogeniertes, also chloriertes oder bromiertes, als auch nichthalogeniertes Naphthalin-1,8-dicarbonsäureimid III eingesetzt werden.

Wird nichthalogeniertes Naphthalin-1,8-dicarbonsäureimid III verwendet, so empfiehlt es sich in der Regel, die Umsetzung bei verschärften Reaktionsbedingungen vorzunehmen, d.h. größere Überschüsse an Naphthalin-1,8-dicarbonsäureimid III und zusätzlich zu einer starken alkalimetallhaltigen Base eine stickstoffhaltige Hilfsbase sowie polar-aprotische Lösungsmittel einzusetzen.

Dementsprechend beträgt das Molverhältnis von Naphthalin-1,8-dicarbonsäureimid III zu Perylen-3,4-dicarbonsäureimid II bei Verwendung von halogeniertem Edukt III (X: Chlor oder Brom) üblicherweise 4 bis 1 : 1 und bevorzugt 2 bis 1 : 1, während es bei nichthalogeniertem Edukt III im allgemeinen bei 8 bis 1 : 1 und vorzugsweise bei 6 bis 2 : 1 liegt.

Als Lösungsmittel sind grundsätzlich alle unter den Reaktionsbedingungen gegen Basen stabilen, hochsiedenden (Siedepunkt > 100°C und oberhalb der gewählten Reaktionstemperatur) Lösungsmittel geeignet, in denen sich die Perylen-3,4-dicarbonsäureimide II und die Naphthalin-1,8-dicarbonsäureimide III bei Reaktionstemperatur vollständig und die verwendeten Basen zumindest partiell lösen, so daß weitgehend homogene Reaktionsbedingungen vorliegen. Besonders geeignet sind unpolar-aprotische und polar-aprotische Lösungsmittel, wobei unpolar-aprotische Lösungsmittel und auf Ethern basierende aprotische Lösungsmittel bei Einsatz von halogenierten Edukten III und die polar-aprotischen Lösungsmittel bei Einsatz von nichthalogenierten Edukten III bevorzugt sind. Es können aber auch protische Lösungsmittel, vorzugsweise solche, die Amino- und Hydroxyfunktionen aufweisen, eingesetzt werden. Selbstverständlich können auch Lösungsmittelgemische verwendet werden.

Beispiele für besonders geeignete unpolar-aprotische Lösungsmittel sind bei > 100°C siedende Lösungsmittel aus den folgenden Gruppen: Aliphaten (insbesondere C₈-C₁₈-Alkane), unsubstituierte, alkylsubstituierte und kondensierte Cycloaliphaten (insbesondere unsubstituierte C₇-C₁₀-Cycloalkane, C₆-C₈-Cycloalkane, die durch ein bis drei C₁-C₆-Alkylgruppen substituiert sind, polycyclische gesättigte Kohlenwasserstoffe mit 10 bis 18 C-Atomen), alkyl- und cycloalkylsubstituierte Aromaten (insbesondere Benzol, das durch ein bis drei C₁-C₆-Alkylgruppen oder einen C₅-C₈-Cycloalkylrest substituiert ist) und kondensierte Aromaten, die alkylsubstituiert und/oder teilhydriert sein können (insbesondere Naphthalin, das durch ein bis vier C₁-C₆-Alkylgruppen substituiert ist) sowie Mischungen dieser Lösungsmittel.

Als Beispiele für bevorzugte unpolar-aprotische Lösungsmittel seien im einzelnen genannt: Octan, Isooctan, Nonan, Isononan, Decan, Isodecan, Undecan, Dodecan, Hexadecan und Octadecan; Cycloheptan, Cyclooctan, Methylcyclohexan, Dimethylcyclohexan, Trimethylcyclohexan, Ethylcyclohexan, Diethylcyclohexan, Propylcyclohexan, Isopropylcyclohexan, Dipropylcyclohexan, Butylcyclohexan, tert.-Butylcyclohexan, Methylcycloheptan und Methylcyclooctan; Toluol, o-, m- und p-Xylol, 1,3,5-Trimethylbenzol (Mesitylen), 1,2,4- und 1,2,3-Trimethylbenzol, Ethylbenzol, Propylbenzol, Isopropylbenzol, Butylbenzol, Isobutylbenzol, tert.-Butylbenzol und Cyclohexylbenzol; Naphthalin, Decahydronaphthalin (Dekalin), 1- und 2-Methylnaphthalin, 1- und 2-Ethylnaphthalin; Kombinationen aus den zuvor genannten Lösungsmitteln, wie sie aus den hochsiedenden, teil- oder durchhydrierten Fraktionen thermischer und katalytischer Crackprozesse bei der Rohöl- oder Naphthaverarbeitung gewonnen werden können, z.B. Gemische vom Exsol^{®} Typ und Alkylbenzolgemische vom Solvesso^{®} Typ.

Besonders bevorzugte unpolar-aprotische Lösungsmittel sind Xylol (alle Isomere), Mesitylen und vor allem Toluol und Dekalin.

Beispiele für besonders geeignete polar-aprotische Lösungsmittel sind N,N-disubstituierte aliphatische Carbonsäureamide (insbesondere N,N-Di-C₁-C₄-alkyl-C₁-C₄-carbonsäureamide), stickstoffhaltige Heterocyclen, Trialkylamine (insbesondere Tri(C₃-C₆-alkyl)amine) und aprotische Ether (insbesondere cyclische Ether, Diarylether und Di-C₁-C₆-alkylether von monomeren und oligomeren C₂-C₃-Alkylenglykolen, die bis zu 6 Alkylenoxideinheiten enthalten können, vor allem Diethylenglykoldi-C₁-C₄-alkylether).

Als Beispiele für bevorzugte polar-aprotische Lösungsmittel seien im einzelnen genannt: N,N-Dimethylformamid, N,N-Diethylformamid, N,N-Dimethylacetamid und N,N-Dimethylbutyramid; N-Methyl-2-pyrrolidon, Chinolin, Isochinolin, Chinaldin, Pyrimidin, N-Methylpiperidin und Pyridin; Tripropyl- und Tributylamin; Di- und Tetramethyltetrahydrofuran, Dioxan, Diphenylether, Diethylenglykoldimethyl-, -diethyl-, -dipropyl-, -diisopropyl-, -di-n-butyl-, -di-sec.-butyl- und -di-tert.-butylether, Diethylenglykolmethylethylether, Triethylenglykoldimethyl- und -diethylether und Triethylenglykolmethylethylether, wobei Diethylenglykoldiethylether, Diphenylether und vor allem Diethylenglykoldimethylether besonders bevorzugt sind.

Besonders geeignete protische Lösungsmittel enthalten Amino- und Hydroxyfunktionen. Beispiele für bevorzugte protische Lösungsmittel sind Alkoholamine, insbesondere Mono-, Di- und Tri-C₂-C₄-alkoholamine, wie Mono-, Di- und Triethanolamin, wobei Ethanolamin besonders bevorzugt ist.

Die Lösungsmittelmenge beträgt in der Regel 50 bis 250 ml unpolar-aprotisches Lösungsmittel, 10 bis 50 ml polar-aprotisches Lösungsmittel bzw. 3 bis 50 ml protisches Lösungsmittel je g Perylen-3,4-dicar-bonsäureimid II.

Als Base sind starke anorganische und organische alkali- oder erdalkalimetallhaltige Basen geeignet, wobei die alkalimetallhaltigen Basen besonders geeignet sind. Bevorzugte anorganische Basen sind Alkali- und Erdalkalimetallhydroxide und -amide, bevorzugte organische Basen sind Alkali- und Erdalkalimetallalkoholate (insbesondere die C₁-C₁₀-Alkoholate, vor allem tert.-C₄-C₆-Alkoholate), Alkali- und Erdalkalimetall-(phenyl)alkylamide (insbesondere die Bis(C₁-C₄-alkyl)amide) und Triphenylmethylmetallate. Besonders bevorzugt sind die Alkalimetallalkoholate. Bevorzugte Alkalimetalle sind Lithium, Natrium und Kalium, wobei Kalium ganz besonders bevorzugt ist. Besonders geeignete Erdalkalimetalle sind Magnesium und Calcium.

Als Beispiele für besonders bevorzugte Basen seien im einzelnen genannt: Lithiumhydroxid, Natriumhydroxid und Kaliumhydroxid; Lithiumamid, Natriumamid und Kaliumamid; Lithiummethylat, Natriummethylat, Kaliummethylat, Lithiumethylat, Natriumethylat, Kaliumethylat, Natriumisopropylat, Kaliumisopropylat, Natrium-tert.-butylat, Kalium-tert.-butylat, Lithium-(1,1-dimethyl)octylat, Natrium-(1,1-dimethyl)octylat und Kalium-(1,1-dimethyl)octylat; Lithiumdimethylamid, Lithiumdiethylamid, Lithiumdiisopropylamid, Natriumdiisopropylamid, Triphenylmethyllithium, Triphenylmethylnatrium und Triphenylmethylkalium. Selbstverständlich können auch Mischungen verschiedener Basen eingesetzt werden.

Ganz besonders bevorzugte Basen sind Lithiumdiisopropylamid, Natriummethylat, Natrium-tert.-butylat, vor allem Kaliummethylat und Kaliumhydroxid und insbesondere Kalium-tert.-butylat.

Bei Verwendung der Methylate und der Hydroxide sowie generell bei Verwendung von nichthalogenierten Edukten III empfiehlt sich zur Erhöhung der Reaktivität der Zusatz einer stickstoffhaltigen Hilfsbase mit geringer nucleophiler Wirkung. Geeignete Basen sind bei den Reaktionstemperaturen flüssige Alkylamine, insbesondere Tri-C₃-C₆-alkylamine, wie Tripropylamin und Tributylamin, Alkoholamine, insbesondere Mono-, Di- und Tri-C₂-C₄-alkoholamine, wie Mono-, Di- und Triethanolamin, und insbesondere heterocyclische Basen, wie Pyridin, N-Methylpiperidin, N-Methylpiperidon, N-Methylmorpholin, N-Methyl-2-pyrrolidon, Pyrimidin, Chinolin, Isochinolin, Chinaldin und vor allem Diazabicyclononen (DBN) und Diazabicycloundecen (DBU). Selbstverständlich können auch Mischungen dieser Hilfsbasen verwendet werden.

Geeignete Einsatzmengen für die Hilfsbase liegen im Fall der halogenierten Edukte III im allgemeinen bei 1 bis 15 g, vorzugsweise bei 1 bis 5 g, je g Perylen-3,4-dicarbonsäureimid II und im Fall der nichthalogenierten Edukte III in der Regel bei 1 bis 60 g, bevorzugt bei 5 bis 30 g, je g Perylen-3,4-dicarbonsäureimid II. Von der Alkalimetallbase werden bei halogenierten Edukten III üblicherweise 2 bis 10 mol, insbesondere 2 bis 4 mol, je mol Perylen-3,4-dicarbonsäureimid II und bei nichthalogenierten Edukten III im allgemeinen 2 bis 20 mol, vorzugsweise 8 bis 20 mol, je mol Perylen-3,4-dicarbonsäureimid II, eingesetzt.

Die Alkalimetallbase kann in fester oder in gelöster Form eingesetzt werden. Wenn die Alkalimetallbase in Kombination mit einem unpolar-aprotischen Reaktionslösungsmittel verwendet wird, in dem sie nicht ausreichend löslich ist, kann sie in einem Alkohol, der eine höhere Basenstärke als die Alkalimetallbase hat, gelöst werden. Geeignet sind vor allem tertiäre aliphatische Alkohole, die Arylsubstituenten enthalten können und insgesamt vier bis zwölf C-Atome aufweisen, z.B. tert.-Butanol, 2-Methyl-2-butanol (tert.-Amylalkohol), 3-Methyl-3-pentanol, 3-Ethyl-3-pentanol, 2-Phenyl-2-pentanol, 2,3-Dimethyl-3-pentanol, 2,4,4-Trimethyl-2-pentanol und 2,2,3,4,4-Pentamethyl-3-pentanol.

Die Reaktionstemperatur liegt üblicherweise bei 50 bis 210°C, bevorzugt bei 70 bis 180°C.

Insbesondere bei Abwesenheit eine Hilfsbase kann es vorteilhaft sein, zunächst eine Reaktionstemperatur im oberen Bereich zu wählen, um das Perylen-3,4-dicarbonsäureimid II in 9-Stellung zu deprotonieren. Die anschließende Kupplungsreaktion mit dem Naphthalin-1,8-dicarbonsäureimid III kann dann in der Regel bei niedrigerer Temperatur durchgeführt werden, was sich insbesondere bei Naphthalin-1,8-dicarbonsäureimiden III mit basenlabilen Substituenten (z.B. Cyclohexyl) am Imidstickstoffatom empfiehlt.

Die Reaktionszeit beträgt in der Regel 1 bis 3 h bei halogenierten Edukten III und 2 bis 8 h bei nichthalogenierten Edukten III.

Verfahrenstechnisch geht man beim Einsatz nichthalogenierter Edukte III zweckmäßigerweise wie folgt vor:

Man legt Perylen-3,4-dicarbonsäureimid II, Naphthalin-1,8-dicarbonsäureimid III und Base vor, gibt Lösungsmittel und gegebenenfalls Hilfsbase unter Schutzgas zu und erhitzt die Mischung die gewünschte Zeit unter Rühren und unter Schutzgas auf die gewünschte Reaktionstemperatur. Nach Abkühlen auf Raumtemperatur fällt man die Terrylen-3,4:11,12-tetracarbonsäurediimide I durch Zugabe von einem protischen Lösungsmittel, das die anderen Komponenten löst, z.B. von C₁-C₃-Alkoholen und insbesondere Wasser, aus. Man filtriert ab und wäscht mit einem der genannten Lösungsmittel, insbesondere mit einem der Alkohole.

Bei Verwendung halogenierter Edukte III kann man analog vorgehen. Man kann jedoch auch zunächst nur ein Gemisch von Perylen-3,4-dicarbonsäureimid II, Base, gegebenenfalls Hilfsbase sowie Lösungsmittel unter Rühren und Schutzgas auf eine Temperatur im Bereich von 120 bis 210°C erhitzen (Deprotonierung) und das Naphthalin-1,8-dicarbonsäureimid III anschließend, gegebenenfalls nach Absenken der Temperatur auf 50 bis 120°C, zugeben.

Gelegentlich kann es zweckmäßig sein, das Reaktionsprodukt einer Oxidation zu unterziehen. Dies kann am einfachsten durch Einblasen von Luftsauerstoff in die noch warme Reaktionsmischung geschehen. Es ist jedoch auch möglich, Oxidationsmittel, wie vorzugsweise Wasserstoffperoxid, aber auch aldehydgruppenhaltige Zucker, z.B. Glukose, insbesondere nach der Reaktion zuzugeben.

Zur weiteren Reinigung kann man die Produkte I z.B. aus einem Gemisch von halogenierten Lösungsmitteln, wie Chloroform und Methylenchlorid, und Alkoholen, wie Methanol, Ethanol und Isopropanol, oder aus einem Carbonsäureamid, wie N-Methylpyrrolidon, umkristallisieren. Alternativ kann man auch eine Säulenchromatographie an Kieselgel unter Verwendung von Methylenchlorid oder Aceton als Eluens vornehmen.

Mit Hilfe des erfindungsgemäßen Verfahrens können die Terrylen-3,4:11,12-tetracarbonsäurediimide I in guten Ausbeuten (in der Regel von 50 bis 80% bei Einsatz halogenierter Edukte III und 25 bis 70% bei Einsatz nichthalogenierter Edukte III) und hohen Reinheiten (üblicherweise 95 bis 99%) auf wirtschaftliche Weise in einem Schritt hergestellt werden. Sowohl an den Imidstickstoffatomen symmetrisch als auch unsymmetrisch substituierte Terrylen-3,4:11,12-tetracarbonsäurediimide I sind auf vorteilhafte Weise zugänglich.

### Beispiele

### Beispiel 1 bis 7

Eine Mischung aus 10 mmol des Perylen-3,4-dicarbonsäureimids II, x ml des Lösungsmittels L und gegebenenfalls b g Diazabicycloundecen (DBU) als Hilfsbase wurde unter Rühren in einer Stickstoffatmosphäre auf T₁°C erwärmt, in 30 min mit insgesamt m mmol der Base B (Beispiel 1 bis 6: B gelöst in 100 ml 2-Methylbutanol; Beispiel 7: B als Feststoff) versetzt. Nach einer Nachrührzeit von t₁ h bei T₁°C und Abkühlen auf T₂°C wurden bei dieser Temperatur portionsweise in 30 min insgesamt 150 ml einer Lösung von 15 mmol (Beispiel 5: 18 mmol) des 4-Bromnaphthalimids III im Lösungsmittel L zugegeben.

Nach einer Nachrührzeit von t₂ h bei T₂°C unter Luft, Abkühlen auf Raumtemperatur und gegebenenfalls Zugabe von 300 ml Methanol zur vollständigen Ausfällung wurde der gebildete Niederschlag abfiltriert, nacheinander mit kaltem Lösungsmittel L, Petrolether und Methanol bis zum farblosen Ablauf gewaschen und bei 100°C im Vakuum getrocknet. Zur weiteren Reinigung wurde das Rohprodukt aus Beispiel 1 bis 4 sowie 6 und 7 einer Säulenchromatographie an Kieselgel mit Methylenchlorid als Eluens und das Rohprodukt aus Beispiel 5 einer fraktionierten Kristallisation aus Schwefelsäure unterzogen.

Weitere Einzelheiten zu diesen Versuchen sowie deren Ergebnisse sind in der folgenden Tabelle zusammengestellt.

Dabei bedeuten:
- IIa:: N-(2,6-Diisopropylphenyl)perylen-3,4-dicarbonsäureimid
- IIb:: N-Methylperylen-3,4-dicarbonsäureimid
- IIc:: N-Cyclohexylperylen-3,4-dicarbonsäureimid
- IIIa:: 4-Brom-N-(2,6-diiisopropylphenyl)naphthalin-1,8-dicarbonsäureimid
- IIIb:: 4-Brom-N-cyclohexylnapthalin-1,8-dicarbonsäureimid
- IIIc:: 4-Brom-N-methylnaphthalin-1,8-dicarbonsäureimid
- B1:: Kalium-tert.-butylat
- B2:: Kaliummethylat
- B3:: Kaliumhydroxid
- DGDME:: Diethylenglykoldimethylether

**Tabelle**

| Bsp. | Edukt II | x [ml] | Lsgm. L | DBU b [g] | T₁ [°C] | m [mmol] | Base B | t₁ [h] | T₂ [°C] | Edukt III | t₂ [h] | Ausbeute [g] / [%] | Reinheit [%] | Smp. [°C] | Aussehen |
|---|---|---|---|---|---|---|---|---|---|---|---|---|---|---|---|
| 1 | IIa | 800 | Dekalin | - | 160 | 40 | B1 | 0,5 | 100 | IIIa | 1 | 4,9/58 | >98 | >300 | schwarzblau, kristallin |
| 2 | IIa | 800 | Dekalin | - | 160 | 40 | B1 | - | 90 | IIIb | 0,5 | 4,9/64 | >98 | >300 | dunkelblau, mikrokristallin |
| 3 | IIa | 800 | Dekalin | 15 | 165 | 25 | B1 | 0,25 | 100 | IIIc | 1 | 4,9/71 | >95 | >300 | schwarzblau, kristallin |
| 4 | IIb | 100 | Diphenyl-ether | 10 | 180 | 30 | B2 | 0,5 | 80 | IIIb | 0,5 | 4,2/68 | >95 | >300 | dunkelblau, amorph |
| 5 | IIb | 75 | DGDME | 10 | 180 | 30 | B1 | 0,25 | 100 | IIIc | 2 | 4,0/73 | >90 | >300 | schwarz, kristallin |
| 6 | IIc | 700 | Dekalin | - | 160 | 40 | B1 | 0,25 | 80 | IIIb | 0,5 | 3,7/55 | >98 | >300 | dunkelblau, amorph |
| 7 | IIc | 50 | DGDME | 12 | 150 | 40 | B3 | 0,5 | 80 | IIIc | 1 | 3,8/62 | >95 | >300 | schwarzblau, mikrokristallin |

### Beispiel 8

10 mmol N-(2,6-Diisopropylphenyl)perylen-3,4-dicarbonsäureimid, 40 mmol N-(2,6-Diisopropylphenyl)naphthalin-1,8-dicarbonsäureimid und 0,2 mol Natrium-tert.-butylat wurden unter Stickstoff in 30 ml Diazabicyclononen (DBN) und 25 ml Diethylenglykoldimethylether gelöst und auf 130°C erwärmt.

Nach dreistündigem Rühren bei dieser Temperatur und Abkühlen auf Raumtemperatur wurde das Reaktionsgemisch auf 100 ml Wasser gegeben. Der Niederschlag wurde abfiltriert und solange mit Ethanol gewaschen, bis das Filtrat rötlich ablief. Nach Umkristallisieren in einem Chloroform/Ethanol-Gemisch wurden 3,5 g blaues Produkt erhalten, was einer Ausbeute von 42% entspricht.

### Beispiel 9

Es wurde analog zu Beispiel 8 vorgegangen, jedoch wurden 0,4 mol Natrium-tert.-butylat und 60 ml DBU sowie anstelle von Diethylenglykoldimethylether 50 ml Ethanolamin eingesetzt, und die Reaktionszeit betrug 6h.

Es wurden 2,3 g Produkt erhalten, was einer Ausbeute von 28% entspricht.

## Patentansprüche

1. Verfahren zur Herstellung von Terrylen-3,4:11,12-tetracarbonsäurediimiden der allgemeinen Formel I 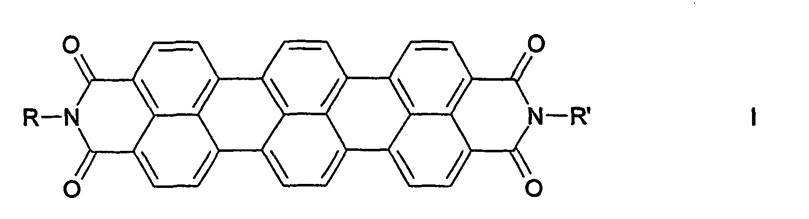 in der die Variablen folgende Bedeutung haben:
R, R' unabhängig voneinander Wasserstoff; C₁-C₃₀-Alkyl, dessen Kohlenstoffkette durch eine oder mehrere Gruppie- rungen -O-, -S-, -NR¹-, -CO- und/oder -SO₂- unterbrochen sein kann und das durch Cyano, C₁-C₆-Alkoxy, Aryl, das durch C₁-C₁₈-Alkyl oder C₁-C₆- Alkoxy substituiert sein kann, und/oder einen über ein Stickstoffatom gebundenen 5- bis 7-gliedrigen heterocyclischen Rest, der weitere Hete- roatome enthalten und aromatisch sein kann, ein- oder mehrfach substituiert sein kann; C₅-C₈-Cycloalkyl, dessen Kohlenstoffgerüst durch eine oder mehrere Gruppierungen -O-, -S- und/oder -NR¹- unterbrochen und/oder das durch C₁-C₆-Alkyl ein- oder mehrfach substituiert sein kann; Aryl oder Hetaryl, das durch C₁-C₁₈-Alkyl, C₁-C₆-Alkoxy, Cyano, Halo- gen, -CONHR² und/oder Aryl- oder Hetarylazo, das jeweils durch C₁-C₁₀- Alkyl, C₁-C₆-Alkoxy oder Cyano substituiert sein kann, ein- oder mehr- fach substituiert sein kann;
R¹ Wasserstoff oder C₁-C₆-Alkyl;
R² Wasserstoff; C₁-C₁₈-Alkyl; Aryl oder Hetaryl, das jeweils durch C₁-C₆- Alkyl, C₁-C₆-Alkoxy, Halogen, Hydroxy, Carboxy oder Cyano substituiert sein kann,
**dadurch gekennzeichnet, daß** man ein Perylen-3,4-dicarbonsäureimid der allgemeinen Formel II 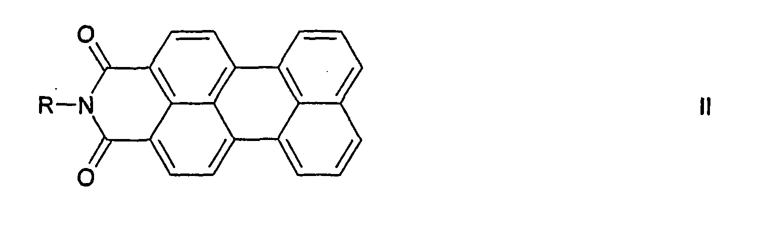 in Gegenwart eines basenstabilen, hochsiedenden, organischen Lösungsmittels und einer alkali- oder erdalkalimetallhaltigen Base mit einem Naphthalin-1,8-dicarbonsäureimid der allgemeinen Formel III 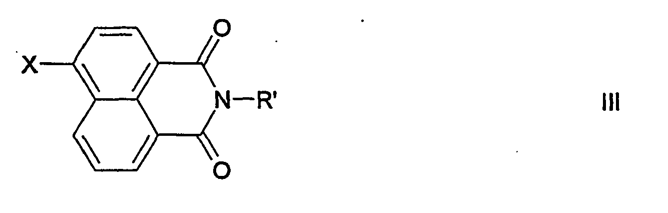 in der X Wasserstoff, Brom oder Chlor bedeutet, umsetzt.

2. Verfahren nach Anspruch 1, **dadurch gekennzeichnet, daß** man als organisches Lösungsmittel ein aprotisches organisches Lösungsmittel einsetzt.

3. Verfahren nach Anspruch 1, **dadurch gekennzeichnet, daß** man als organisches Lösungsmittel ein polar-aprotisches organisches Lösungsmittel einsetzt.

4. Verfahren nach Anspruch 1, **dadurch gekennzeichnet, daß** man als organisches Lösungsmittel ein unpolar-aprotisches Lösungsmittel einsetzt.

5. Verfahren nach Anspruch 1, **dadurch gekennzeichnet, daß** man als organisches Lösungsmittel ein protisches Lösungsmittel einsetzt.

6. Verfahren nach Anspruch 1, **dadurch gekennzeichnet, daß** man als organisches Lösungsmittel ein Amino- und Hydroxyfunktionen enthaltendes Lösungsmittel einsetzt.

7. Verfahren nach den Ansprüchen 1 bis 6, **dadurch gekennzeichnet, daß** man als Base eine starke anorganische oder organische alkalimetallhaltige Base einsetzt.

8. Verfahren nach den Ansprüchen 1 bis 7, **dadurch gekennzeichnet, daß** man als Base ein Alkalimetallalkoholat einsetzt.

9. Verfahren nach den Ansprüchen 1 bis 8, **dadurch gekennzeichnet, daß** man eine stickstoffhaltige Base mit geringer nucleophiler Wirkung zusätzlich als Hilfsbase einsetzt.

10. Verfahren nach den Ansprüchen 1 bis 9, **dadurch gekennzeichnet, daß** man die Umsetzung bei 50 bis 210°C vornimmt.

## Claims

1. A process for preparing a terylene-3,4:11,12-tetracarboximides of the general formula I 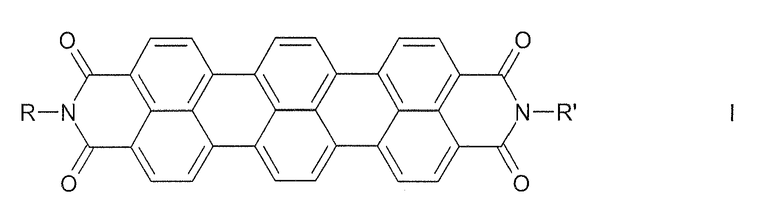 in which the variables are each defined as follows:
R, R' are each independently hydrogen; C₁-C₃₀-alkyl whose carbon chain may be interrupted by one or more -O-, -S-, -NR¹-, -CO- and/or -SO₂- moieties and which may be mono- or polysubstituted by cyano, C₁-C₆-alkoxy, aryl which may be substituted by C₁-C₁₈-alkyl or C₁-C₆-alkoxy, and/or a 5- to 7-membered heterocyclic radical bonded via a nitrogen atom which may comprise further heteroatoms and be aromatic; C₅-C₈-cycloalkyl whose carbon skeleton may be interrupted by one or more -O-, -S- and/or -NR¹- moieties, and/or which may be mono- or polysubstituted by C₁-C₆-alkyl; aryl or hetaryl which may be mono- or polysubstituted by C₁-C₁₈-alkyl, C₁-C₆-alkoxy, cyano, halogen, -CONHR² and/or aryl- or hetarylazo, each of which may be substituted by C₁-C₁₀-alkyl, C₁-C₆-alkoxy or cyano;
R¹ is hydrogen or C₁-C₆-alkyl;
R² is hydrogen, C₁-C₁₈-alkyl; aryl or hetaryl, each of which may be substituted by C₁-C₆-alkyl, C₁-C₆-alkoxy, halogen, hydroxyl, carboxyl or cyano,
which comprises reacting a perylene-3,4-dicarboximide of the general formula II 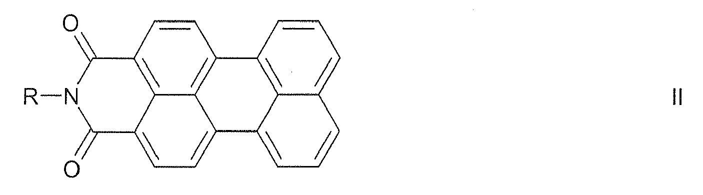 in the presence of a base-stable, high-boiling organic solvent and of an alkali metal or alkaline earth metal base, with a naphthalene-1,8-dicarboximide of the general formula III 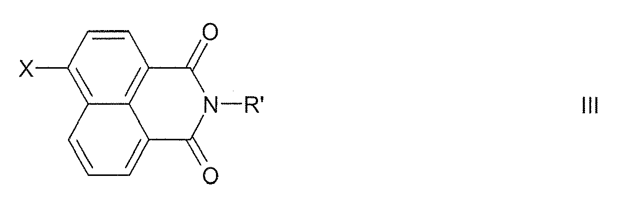 in which X is hydrogen, bromine or chlorine.

2. The process according to claim 1, wherein the organic solvent used is an aprotic organic solvent.

3. The process according to claim 1, wherein the organic solvent used is a polaraprotic organic solvent.

4. The process according to claim 1, wherein the organic solvent used is a nonpolar-aprotic organic solvent.

5. The process according to claim 1, wherein the organic solvent used is a protic organic solvent.

6. The process according to claim 1, wherein the organic solvent used is a solvent comprising amino and hydroxyl functions.

7. The process according to claims 1 to 6, wherein the base used is a strong inorganic or organic alkali metal base.

8. The process according to claims 1 to 7, wherein the base used is an alkali metal alkoxide.

9. The process according to claims 1 to 8, wherein a nitrogen base having lesser nucleophilic action is additionally used as an auxiliary base.

10. The process according to claims 1 to 9, wherein the reaction is undertaken at from 50 to 210°C.

## Revendications

1. Procédé de fabrication de diimides de l'acide térylène-3,4:11,12-tétracarboxylique de formule générale I 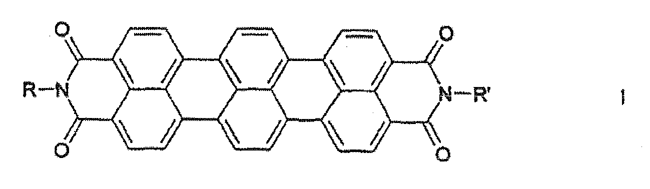 dans laquelle les variables ont la signification suivante :
R, R' indépendamment l'un de l'autre, l'hydrogène ; un alkyle en C₁-C₃₀, dont la chaîne carbonée peut être interrompue par un ou plusieurs groupes -O-, -S-, -NR¹-, -CO- et/ou -SO₂- et qui peut être substitué une ou plusieurs fois par un cyano, un alcoxy en C₁-C₆, un aryle, qui peut être substitué par un alkyle en C₁-C₁₈ ou un alcoxy en C₁-C₆, et/ou un radical hétérocyclique ayant 5 à 7 éléments relié par un atome d'azote, qui peut contenir des hétéroatomes supplémentaires et être aromatique ; un cycloalkyle en C₅-C₈, dont le squelette carboné peut être interrompu par un ou plusieurs groupes -O-, -S- et/ou -NR¹- et/ou qui peut être substitué une ou plusieurs fois par un alkyle en C₁-C₆ ; un aryle ou un hétaryle, qui peut être substitué une ou plusieurs fois par un alkyle en C₁-C₁₈, un alcoxy en C₁-C₆, un cyano, un halogène, -CONHR²- et/ou un aryl- ou hétarylazo, qui peut à chaque fois être substitué par un alkyle en C₁-C₁₀, un alcoxy en C₁-C₆ ou un cyano ;
R¹ l'hydrogène ou un alkyle en C₁-C₆ ;
R² l'hydrogène ; un alkyle en C₁-C₁₈ ; un aryle ou un hétaryle, qui peut à chaque fois être substitué par un alkyle en C₁-C₆, un alcoxy en C₁-C₆, un halogène, un hydroxy, un carboxy ou un cyano,
**caractérisé en ce qu'**un imide de l'acide pérylène-3,4-dicarboxylique de formule générale II 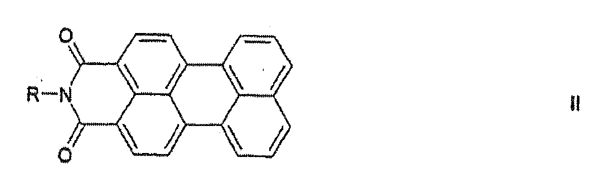 est mis en réaction, en présence d'un solvant organique de point d'ébullition élevé, stable en milieu basique, et d'une base contenant un métal alcalin ou alcalino-terreux, avec un imide de l'acide naphtaline-1,8-dicarboxylique de formule générale III 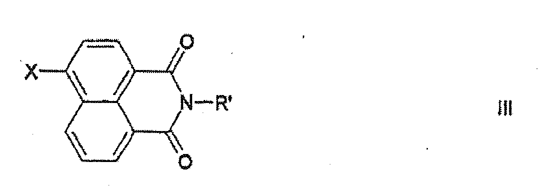 dans laquelle X représente l'hydrogène, le brome ou le chlore.

2. Procédé selon la revendication 1, **caractérisé en ce qu'**un solvant organique aprotique est utilisé en tant que solvant organique.

3. Procédé selon la revendication 1, **caractérisé en ce qu'**un solvant organique aprotique polaire est utilisé en tant que solvant organique.

4. Procédé selon la revendication 1, **caractérisé en ce qu'**un solvant aprotique apolaire est utilisé en tant que solvant organique.

5. Procédé selon la revendication 1, **caractérisé en ce qu'**un solvant protique est utilisé en tant que solvant organique.

6. Procédé selon la revendication 1, **caractérisé en ce qu'**un solvant contenant des fonctions amino et hydroxy est utilisé en tant que solvant organique.

7. Procédé selon les revendications 1 à 6, **caractérisé en ce qu'**une base forte inorganique ou organique contenant un métal alcalin est utilisée en tant que base.

8. Procédé selon les revendications 1 à 7, **caractérisé en ce qu'**un alcoolate de métal alcalin est utilisé en tant que base.

9. Procédé selon les revendications 1 à 8, **caractérisé en ce qu'**une base contenant de l'azote ayant un pouvoir nucléophile faible est également utilisée en tant que base auxiliaire.

10. Procédé selon les revendications 1 à 9, **caractérisé en ce que** la réaction est réalisée à 50 à 210°C.
